# FASCICULE DE BREVET EUROPEEN

(11) **EP 4 225 294 B1**
(45) Date de publication et mention de la délivrance du brevet: **04.12.2024**
(21) Numéro de dépôt: 21783026.4
(22) Date de dépôt: 07.10.2021
(51) Int. Cl.: A61K 31/198, A61P 3/10

(54) **O-ACÉTYLSÉRINE POUR SON UTILISATION DANS LA PRÉVENTION ET LE TRAITEMENT DE L'INTOLÉRANCE AU GLUCOSE ET LES MALADIES ASSOCIÉES**
O-ACETYLSERIN ZUR VERWENDUNG BEI DER PRÄVENTION UND BEHANDLUNG VON GLUCOSEINTOLERANZ UND DAMIT ASSOZIIERTEN ERKRANKUNGEN
O-ACETYLSERINE FOR THE USE THEREOF IN THE PREVENTION AND TREATMENT OF GLUCOSE INTOLERANCE AND THE ASSOCIATED DISEASES

(30) Priorité: 07.10.2020 FR 2010231
(43) Date de publication de la demande: 16.08.2023
(73) Titulaire: Institut national de recherche pour l'agriculture, l'alimentation et l'environnement, 75007 Paris (FR); Institut National des Sciences et Industries du Vivant et de l'Environnement, 91120 Palaiseau (FR); Université Paris-Saclay, 91190 Gif-sur-Yvette (FR)
(72) Inventeur: DELORME, Christine, 91120 PALAISEAU (FR); DOUARD, Véronique, 91440 BURES-SUR-YVETTE (FR)
(74) Mandataire: Cabinet Nony
(86) Numéro de dépôt international: PCT/EP2021/077779
(87) Numéro de publication internationale: WO 2022/074154

(56) Documents cités:
- EP-A1- 1 233 067

## Description

### Domaine technique

La présente invention concerne le domaine de la prévention et/ou du traitement de désordres métaboliques tels que l'intolérance au glucose ou prédiabète, ainsi que de maladies liées à l'intolérance au glucose, tel que le Diabète de Type 2.

### Technique antérieure

Le Diabète de Type 2 (DT2) est une maladie caractérisée par une hyperglycémie chronique, résultant de l'utilisation inadéquate de l'insuline par l'organisme. Cette maladie est généralement la conséquence d'un excès pondéral et de l'inactivité physique, comme, par exemple, l'obésité qui se développe en réponse à des changements nutritionnels et de mode de vie. Ainsi, les facteurs de risque associés au DT2 sont notamment : le surpoids, l'âge avancé, une mauvaise alimentation et un excès de calories ou une mauvaise nutrition, l'inactivité physique, le tabagisme ou encore les antécédents familiaux (Vazquez et al. Comparison of body mass index, waist circumference, and waist/hip ratio in predicting incident diabètes: a meta-analysis. Epidemiol Rev 2007; 29: 115-28; Diabètes Prévention Program Research Group. Long-term effects of lifestyle intervention or metformin on diabètes development and microvascular complications over 15-year follow-up: the Diabètes Prévention Program Outcomes Study. Lancet Diabètes Endocrinol 2015; 3: 866-75).

Le DT2 est le diabète le plus répendu dans le monde avec 90% de tous les cas (World Health Organization. 1999. Définition, diagnosis and classification of diabètes mellitus and its complications: report of a WHO consultation. Part 1, Diagnosis and classification of diabètes mellitus). Le diabète est un réel problème de santé publique du fait de sa prévalence mondiale qui a doublé entre 1980 et 2014 passant de 4,7 à 8,5 % de la population adulte (Rapport mondial sur le diabète, Organisation mondiale de la Santé 2016, ISBN: 9789242565256), du coût élevé du traitement et de ses complications. En effet, le diabète, à long terme, peut affecter le coeur, les vaisseaux sanguins, les yeux, les reins et les nerfs, et accroître le risque de cardiopathie et d'infarctus du myocarde. Par conséquent, prévenir et traiter le DT2, est un champ d'investigation thérapeutique hautement pertinent avec de forts enjeux économiques et sociétaux (Atlas du diabète de la FID - Sème Édition, 2017).

De nos jours, les recommandations de mesures hygiéno-diététiques ciblent le prédiabète et, bien que peu prescrits, des traitements préventifs sont recommandés pour des personnes à fort risque de développer un diabète de type 2. Le prédiabète, phase précoce et réversible du diabète, se caractérise, notamment, par une intolérance au glucose.

L'intolérance au glucose est un facteur de risque du diabète de type 2, qui s'installe dans la phase de prédiabète. L'intolérance au glucose ou prédiabète ou hyperglycémie intermédiaire est un trouble de la glycémie. Un individu souffrant d'intolérance au glucose présente une glycémie supérieure à la normale mais qui n'est pas assez élevée pour poser un diagnostic de diabète. Il est possible de caractériser une intolérance au glucose par la détection d'une diminution de la sensibilité à l'insuline ou une augmentation de la résistance à l'insuline. Le prédiabète est une phase au cours de laquelle il est souvent encore possible d'inverser la tendance diabétique pour revenir à une glycémie normale. Cet état précurseur du diabète de type 2 peut être, dans certains cas, réversible avec de l'exercice physique et une adaptation du régime alimentaire.

De manière générale, l'activité physique associée à un régime équilibré peut permettre de diminuer le glucose sanguin et prévenir la progression vers le DT2. Cependant l'augmentation prévisionnelle de cas de diabète de type 2 dans les prochaines années suggère que ces aménagements de style de vie ne sont pas suffisants. Ainsi, il apparaît qu'un rééquilibrage de l'hygiène de vie est souvent peu réaliste ou insuffisant et peut être associé à une médication orale prescrite pour contrôler le niveau de glucose sanguin. Toutefois, ces médications sont peu nombreuses et certaines ne sont pas conseillées chez les enfants et adolescents qui sont de plus en plus nombreux à développer une intolérance au glucose. Par exemple, la Metformine, généralement utilisée pour traiter le DT2, peut être prescrite. De plus, des contre-indications et des effets secondaires indésirables existent, tels que des nausées, vomissements, diarrhées, douleurs abdominales ou perte de l'appétit. Ces effets indésirables sont la cause d'arrêts fréquents de traitements (McGovern et al. Comparison of médication adhérence and persistence in type 2 diabètes: A systematic review and meta-analysis. Diabètes Obesity and Metabolism, 2017, 20(4):1040-1043). Un traitement à la Metformine sur le long terme est associé à une augmentation du risque de carences en vitamine B6 et B12, qui peut s'accompagner de complications neurologiques (Porter et al. Hyperglycemia and Metformin Use Are Associated With B Vitamin Deficiency and Cognitive Dysfunction in Older Adults. J. Clin. Endocrinol. Metab., 2019, 104(10): 4837-4847).

Cibler l'état prédiabétique, réversible, et en particulier l'intolérance au glucose, de manière précoce, apparaît donc être essentiel pour prévenir son évolution vers le diabète de type 2.

Il existe un besoin de disposer de nouveaux composés ou compositions pour contrôler l'homéostasie du glucose. Il existe également un besoin de disposer de nouveaux composés ou compositions permettant de cibler l'intolérance au glucose.

Il existe un besoin de disposer de nouveaux composés ou compositions dont l'innocuité peut convenir à une administration chez l'enfant ou l'adolescent.

Il existe un besoin de disposer de nouveaux traitements pour prévenir et/ou traiter l'intolérance au glucose. Il existe également un besoin de disposer de nouveaux traitements pour prévenir et/ou traiter les maladies associées ou résultant d'une intolérance au glucose, comme, notamment, le diabète de type 2. Ainsi, il existe un besoin de disposer de nouveaux composés ou compositions pour prévenir et/ou traiter le prédiabète.

Il existe ainsi également un besoin pour prévenir et/ou traiter l'hyperglycémie postprandiale. Il existe un besoin pour traiter et/ou prévenir les maladies associées à une intolérance au glucose, en particulier le diabète de type 2.

La présente invention a pour objet de satisfaire tout ou partie de ces besoins.

### Exposé de l'invention

La présente invention propose un métabolite de synthèse, l'O-acétylsérine, pour son utilisation dans le traitement et/ou la prévention d'une intolérance au glucose. Elle propose également l'O-actéylsérine, pour son utilisation dans le traitement et/ou la prévention d'une ou plusieurs maladies liées à une intolérance au glucose, et notamment du Diabète de Type 2.

### Résumé de l'invention

L'objectif de la présente invention est de proposer de nouvelles substances pour leur utilisation dans une méthode thérapeutique pour prévenir et/ou traiter une intolérance au glucose et/ou une maladie associée à une intolérance au glucose chez un individu.

De manière surprenante, les inventeurs ont observé un effet protecteur de l'O-acétylsérine (ou OAS) sur l'homéostasie du glucose. L'OAS limite la mise en place de l'intolérance au glucose induit par des régimes alimentaires obésogènes (riches en graisses ou en sucres) et qui précède l'installation du DT2. La prévention et le traitement de l'intolérance au glucose permettent ainsi la prévention et le traitement du DT2.

Ainsi, selon un premier aspect, l'invention concerne l'O-acétylsérine, ou l'un de ses sels pour son utilisation dans le traitement et/ou la prévention de l'intolérance au glucose et/ou d'une ou plusieurs maladies associées à une intolérance au glucose chez un individu.

Dans un mode de réalisation particulier, la maladie associée à une intolérance au glucose est le diabète de type 2.

En outre, l'individu peut être en surpoids ou obèse.

Dans un mode de réalisation particulier, l'O-acétylsérine est un métabolite de synthèse de bactéries, en particulier est un métabolite de synthèse de bactéries du microbiote intestinale, plus particulièrement est un métabolite de synthèse de la souche bactérienne *Streptococcus salivarius.*

Selon un second aspect, l'invention concerne une composition pour son utilisation dans le traitement et/ou la prévention de l'intolérance au glucose et/ou d'une ou plusieurs maladies associées à l'intolérance au glucose chez un individu comprenant une O-acétylsérine, ou l'un de ses sels et un véhicule physiologiquement acceptable.

Dans un mode de réalisation particulier, la maladie associée à une intolérance au glucose est le diabète de type 2.

En outre, l'individu peut être obèse.

Selon un mode de réalisation, l'O-acétylsérine est un métabolite de synthèse de bactéries, en particulier est un métabolite de synthèse de bactéries du microbiote intestinale, plus particulièrement est un métabolite de synthèse de la souche bactérienne *Streptococcus salivarius.*

Selon l'invention, la composition peut être une composition pour voie orale, en particulier sous la forme d'une poudre, de granulés, d'un produit alimentaire, d'une boisson, d'un produit pharmaceutique, d'un nutraceutique, d'un additif alimentaire, d'un complément alimentaire, d'une capsule ou d'une gélule.

Dans un mode de réalisation particulier, la composition comprend en outre un actif additionnel, en particulier choisi parmi des métabolites, des antioxydants, des huiles de poisson, DHA, EPA, des vitamines, des minéraux, des phytonutriments, une protéine, un lipide, des probiotiques, des actifs additionnels permettant de prévenir et/ou traiter une intolérance au glucose, et des combinaisons de ceux-ci.

### Brève description des dessins

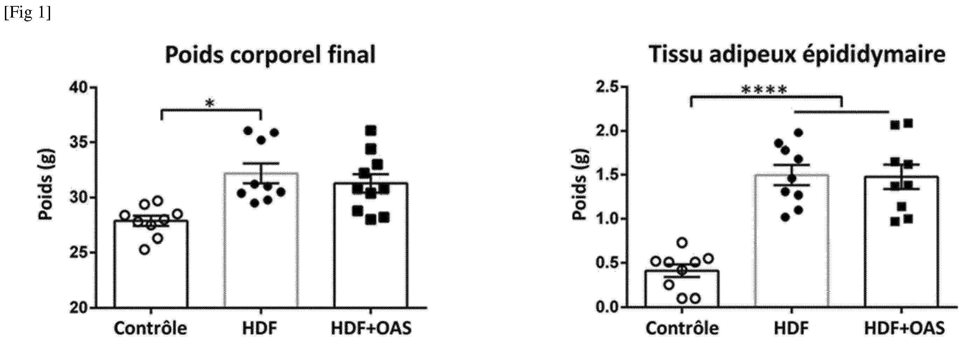

[Fig 1] représente le poids corporel final (gauche) et le poids du tissu adipeux épididymaire des groupes de souris étudiées après 12 semaines de régime et traitement : le groupe contrôle ayant reçu un régime contrôle, le groupe HFD ayant reçu un régime riche en graisses (High Fat Diet) non traité (HFD), et le groupe HFD traité avec de l'O-acétylsérine (HFD+OAS). Le schéma de gauche représente le poids moyen des souris de chacun des groupes en grammes (ordonnées). Les groupes de souris, de gauche à droite, sont indiqués sur l'axe des abscisses : Contrôle ; HFD et HFD+OAS. La différence de poids entre le groupe contrôle et le groupe HFD est statistiquement significatif (* p<0.05).

Le schéma de droite représente le poids moyen du tissu adipeux épididymaire des souris de chacun des groupes en grammes (ordonnées). Les groupes de souris, de gauche à droite, sont indiqués sur l'axe des abscisses : Contrôle ; HFD et HFD+OAS. La différence de poids du tissu adipeux épididymaire entre le groupe contrôle et le groupe HFD ou le groupe HFD+OAS est statistiquement significatif (**** p<0.0001).

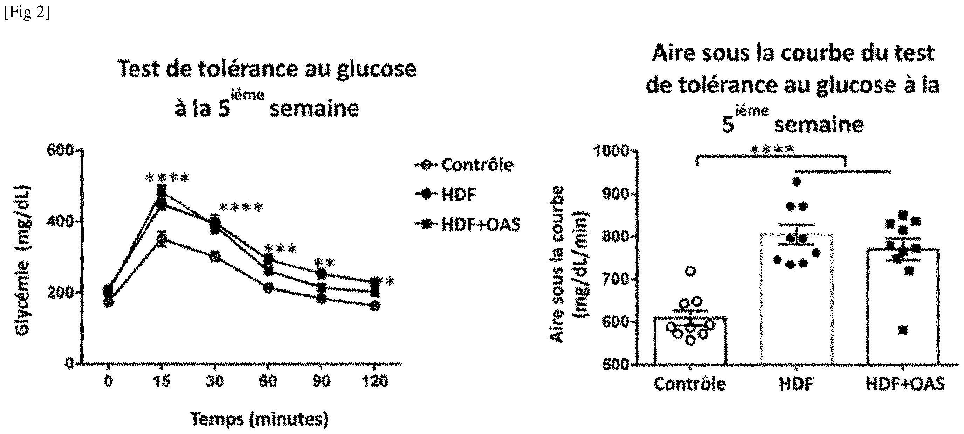

[Fig 2] représente les résultats du test de tolérance au glucose après 5 semaines d'étude pour chacun des groupes de souris étudiées : le groupe contrôle ayant reçu un régime contrôle, le groupe HFD ayant reçu un régime riche en graisses (High Fat Diet) non traité (HFD), et le groupe HFD traité avec de l'O-acétylsérine (HFD+OAS). A gauche, les courbes représentent la variation de la concentration de glucose moyenne dans le plasma des souris en mg/dl (ordonnées) en fonction du temps en minutes (abscisses) pour chacun des groupes de souris après une charge orale de glucose. Le groupe contrôle est représenté par la courbe comprenant le symbole o, le groupe HFD est représenté par la courbe ayant le symbole ● et le groupe HFD+OAS est représenté par la courbe ayant le symbole ■. La différence entre d'un côté le groupe contrôle et de l'autre côté les groupes HFD et HFD+OAS est significative (avec, respectivement ****p<0.0001, ***p<0.001, **p<0.01 et *p<0.05 à t=15mn et à t=30mn, à t=60mn, à t=90mn et à t=120mn).

A droite, les colonnes représentent l'aire sous la courbe en mg/dl/min (ordonnées) à partir des courbes du schéma de gauche pour chacun des groupes de souris indiqués, de gauche à droite, sur l'axe des abscisses : Contrôle ; HFD et HFD+OAS. La différence dans l'aire sous la courbe entre d'un côté le groupe contrôle et de l'autre côté les groupes HFD et HFD+OAS est significative (****p<0.0001).

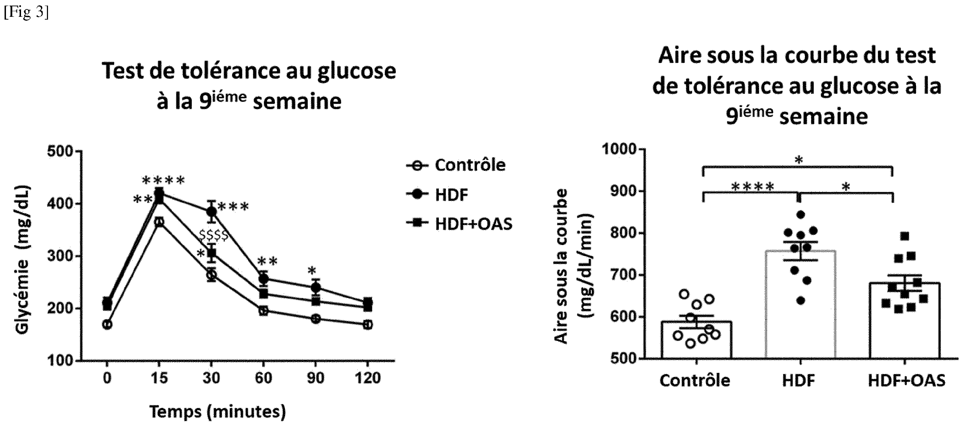

[Fig 3] représente les résultats du test de tolérance au glucose après 9 semaines d'étude pour chacun des groupes de souris étudiées : le groupe contrôle ayant reçu un régime contrôle, le groupe HFD ayant reçu un régime riche en graisses (High Fat Diet) non traité (HFD), et le groupe HFD traité avec de l'O-acétylsérine (HFD+OAS). A gauche, les courbes représentent la variation de la concentration de glucose moyenne dans le plasma des souris en mg/dl (ordonnées) en fonction du temps en minutes (abscisses) pour chacun des groupes de souris après une charge orale de glucose. Le groupe contrôle est représenté par la courbe comprenant le symbole o, le groupe HFD est représenté par la courbe ayant le symbole • et le groupe HFD+OAS est représenté par la courbe ayant le symbole ■. La différence entre d'un côté le groupe contrôle et de l'autre côté les groupes HFD et HFD+OAS est significative (avec, respectivement ****p<0.0001, ***p<0.001, **p<0.01, et *p<0.05 à t=15mn, à t=30mn, à t=60mn, et à t=90mn). A partir de t=60mn, la différence entre d'un côté le groupe contrôle et de l'autre côté le groupe HFD+OAS n'est plus significative et à t=30 mn, la différence entre d'un côté le groupe HFD et de l'autre côté le groupe HFD+OAS est significative ^{$$$$}p<0.0001.

A droite, les colonnes représentent l'aire sous la courbe en mg/dl/min (ordonnées) à partir des courbes du schéma de gauche pour chacun des groupes de souris indiqués, de gauche à droite, sur l'axe des abscisses : Contrôle ; HFD et HFD+OAS. La différence dans l'aire sous la courbe entre chacun des groupes de souris est significative (****p<0.0001 entre le groupe contrôle et le groupe HFD, *p<0.05 entre le groupe HFD et le groupe HFD+OAS et *p<0.05 entre le groupe contrôle et le groupe HFD+OAS).

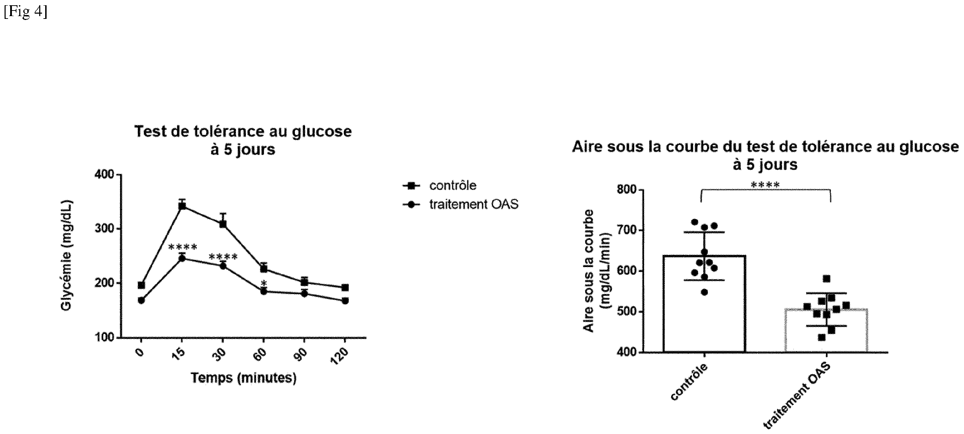

[Fig 4] représente les résultats du test de tolérance au glucose après 5 jours d'étude pour chacun des groupes de souris étudiées : le groupe contrôle a reçu un régime alimentaire classique et n'a pas reçu d'O-acétylsérine, tandis que le groupe OAS a également reçu un régime alimentaire classique ainsi qu'une dose journalière d'O-acétylsérine (OAS).

A gauche, les courbes représentent la variation de la concentration de glucose moyenne dans le plasma des souris en mg/dL (ordonnées) en fonction du temps en minutes (abscisses) pour chacun des groupes de souris après une charge orale de glucose à t0. Le groupe contrôle est représenté par la courbe comprenant le symbole ■, le groupe OAS est représenté par la courbe ayant le symbole •. La différence entre le groupe contrôle et le groupe OAS est significative (avec, respectivement ****p<0.0001 et *p<0.05 à t=15mn, à t=30mn et à t=60mn).

A droite, les colonnes représentent l'aire sous la courbe en mg/dL/min (ordonnées) à partir des courbes du schéma de gauche pour chacun des groupes de souris indiqués, de gauche à droite, sur l'axe des abscisses : Contrôle ; OAS. La différence dans l'aire sous la courbe entre chacun des groupes de souris est significative (****p<0.0001).

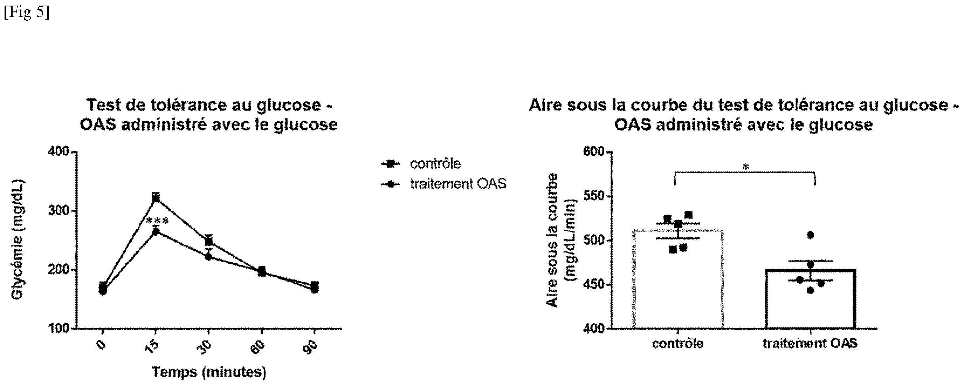

[Fig 5] représente les résultats du test de tolérance au glucose après réception pour chacun des groupes de souris étudiées d'une charge orale de glucose : le groupe contrôle a reçu que la charge orale de glucose à t0, tandis que le groupe OAS a reçu une dose d'O-acétylsérine (OAS) en même temps que la charge orale de glucose à t0.

A gauche, les courbes représentent la variation de la concentration de glucose moyenne dans le plasma des souris en mg/dL (ordonnées) en fonction du temps en minutes (abscisses) pour chacun des groupes de souris après une charge orale de glucose à t0. Le groupe contrôle est représenté par la courbe comprenant le symbole ■, le groupe OAS est représenté par la courbe ayant le symbole •. La différence entre le groupe contrôle et le groupe OAS est significative (avec ***p<0.001 à t=15mn).

A droite, les colonnes représentent l'aire sous la courbe en mg/dL/min (ordonnées) à partir des courbes du schéma de gauche pour chacun des groupes de souris indiqués, de gauche à droite, sur l'axe des abscisses : Contrôle ; OAS. La différence dans l'aire sous la courbe entre chacun des groupes de souris est significative (*p<0.05).

### Description détaillée

Les inventeurs ont conduit des travaux approfondis afin d'identifier la capacité de l'O-acétylsérine à traiter et/ou prévenir une intolérance au glucose et/ou une maladie associée à une intolérance au glucose chez un individu en ayant besoin. En particulier, les inventeurs ont démontré la capacité de l'OAS à traiter et/ou prévenir une intolérance au glucose chez la souris.

En effet, comme développé dans la partie expérimentale ci-dessous, les inventeurs ont démontré qu'un traitement avec de l'OAS permet de réguler le métabolisme du glucose avec notamment une amélioration de l'intolérance au glucose, en particulier l'intolérance au glucose associée à l'obésité, dans un modèle de souris soumis à un régime alimentaire obésogène. Ils ont ainsi démontré l'utilité de l'OAS pour améliorer *in vivo* des dérèglements métaboliques, notamment provoqués par l'obésité.

### Définitions

Les termes utilisés dans la présente description sont utilisés avec leur signification habituelle dans le domaine technique considéré, et au regard du contexte de la description dans lequel les termes sont utilisés. Certains termes sont d'avantage discutés ci-dessous, ou ailleurs dans la description, pour fournir des indications supplémentaires au regard de l'invention et de sa mise en oeuvre. Les définitions qui suivent sont fournies pour la description et les revendications.

La description des différents modes de réalisation de l'invention comprend les modes de réalisation incluant « **comprenant** », « **ayant** », « **consistant en** » et « **consistant essentiellement en** ». Les mots « **avoir** » et « **comprendre** », ou des variantes telles que « **a** », « **ont** », « **comprend** » ou « **comprenant** » doivent être compris comme impliquant l'inclusion du ou des éléments indiqués (comme un élément d'une composition ou une étape de méthode) mais pas l'exclusion d'autres éléments. Le terme « consistant en » implique l'inclusion du ou des éléments indiqués, à l'exclusion de tout élément supplémentaire. L'expression « **consistant essentiellement en** » implique l'inclusion des éléments indiqués, et éventuellement d'autres éléments lorsque les autres éléments n'affectent pas matériellement les caractéristiques fondamentales et nouvelles de l'invention. Selon le contexte, le terme « comprendre » peut également indiquer strictement les caractéristiques indiquées, les nombres entiers, les étapes ou les composants indiqués et, par conséquent, dans ce cas, il peut être remplacé par « consister en ».

Par « **diabète de type 2** » ou « **DT2** », on entend désigner une maladie chronique qui se déclare lorsque le pancréas ne produit pas suffisamment d'insuline (hormone régulatrice de la glycémie), ou lorsque l'organisme n'est pas capable d'utiliser efficacement l'insuline qu'il produit. Un DT2 est généralement précédé d'une intolérance au glucose.

Les termes « **environ** » ou « **approximativement** » tels qu'utilisés ici au regard d'une valeur numérique se réfèrent à la plage d'erreur habituelle pour la valeur considérée, telle qu'elle habituellement identifiée par l'homme du métier dans le domaine technique considéré. La mention du terme « environ » au regard d'une valeur ou d'un paramètre spécifique inclut et décrit en tant que tel cette valeur ou ce paramètre. Le terme « environ » fait référence à ± 10% d'une valeur donnée. Cependant, chaque fois que la valeur en question se réfère à un objet indivisible qui perdrait son identité une fois subdivisé, alors « environ » fait référence à ± 1 de l'objet indivisible.

Le terme « **individu** » ou « **patient** » tel qu'utilisé dans le présent texte désigne en particulier un mammifère. Les mammifères considérés incluent les, mais ne sont pas limités aux, animaux domestiques (par exemple, bovins, ovins, chats, chiens, et chevaux), les primates (par exemple humains et non-humains), les lapins et les rongeurs (par exemple, les souris et les rats). Selon un mode de réalisation particulier, un individu, ou patient, est un être humain. Par « **intolérance au glucose** » on entend désigner un état physiopathologique dans lequel la glycémie d'un individu est supérieure à la normale mais inférieure au seuil de diagnostic du diabète de type 2. Une intolérance au glucose peut être détectée par une mesure de glycémie à jeun et par le suivi de la glycémie au cours d'un test oral de tolérance au glucose (ou test OGTT).

Par « **obésité** », on entend désigner un état physiopathologique dans lequel un individu présente, notamment, un excès de tissu adipeux, généralement induit par un régime obésogène, comprenant, notamment, une consommation excessive d'aliments caloriques, des prédispositions génétiques ou une pratique sportive insuffisante ou inexistante. Un individu déclaré comme obèse possède un indice de masse corporel (IMC) supérieur à 30. L'indice de masse corporel, selon une définition officielle de l'Organisme Mondiale de la Santé (OMS), est l'indicateur des risques pour la santé associée à un poids excessif et à un poids insuffisant. L'IMC se calcule en divisant le poids de l'individu (en kilogrammes) par sa taille (en mètres) élevée au carré. Une valeur d'IMC est associée à une corpulence spécifique selon la classification donnée par l'OMS.

A la différence d'un individu « **obèse** », on entend par un « **individu en surpoids** » un individu dont l'état n'est pas physiopathologique. Un individu en surpoids présente souvent également un excès de tissu adipeux. On considère généralement qu'un individu est en surpoids lorsqu'il possède un indice de masse corporel (IMC) compris entre 25 et 30.

Par « **prédiabète** », on entend désigner un état physiopathologique caractérisé, notamment, par une glycémie élevée par rapport à la normale, mais inférieure au seuil de définition du diabète de type 2. Une glycémie à jeun est considérée comme étant (i) normale entre 0.70 et 1.10 g/l, (ii) signe de prédiabète entre 1.10 et 1.25 g/l et (iii) signe de diabète quand > 1.25 g/l. Le prédiabète n'induit généralement pas de symptômes, mais est souvent associé à une obésité, une dyslipidémie, et de l'hypertension. C'est un facteur de risque de maladies cardiovasculaires. Le prédiabète se caractérise, notamment, par une intolérance au glucose. Dans le contexte de la présente invention, les termes « **prévenir** », « **prévention** » et « **ralentir la progression de** » (et les variantes de ces expressions) au regard d'un désordre physiologique ou d'une maladie concerne le traitement prophylactique de la maladie ou du désordre, par exemple chez un individu suspecté d'avoir cette maladie ou ce désordre, ou étant à risque de développer cette maladie ou ce désordre. Prévenir inclut, mais n'est pas limité à, la prévention ou le ralentissement du développement de la maladie, et/ou le maintien d'un ou plusieurs symptômes de maladie à un niveau désiré ou un niveau moindre. Le terme « prévenir » ne requiert pas l'élimination à 100 % de la possibilité ou de la probabilité de survenue de la maladie ou du désordre. Ce terme désigne plutôt la réduction à un degré moindre du risque ou de la probabilité d'occurrence d'un phénomène donné, c'est-à-dire, dans la présente invention, d'une intolérance au glucose ou d'une maladie associée à une intolérance au glucose, telle qu'un état prédiabétique ou un diabète de type 2. Comme indiqué, la prévention peut être complète, c'est-à-dire l'absence de symptômes ou de maladie détectable, ou partielle, de telle qu'il y ait moins de symptômes ou que les symptômes soient d'intensité moindre.

Par « **homéostasie du glucose** », on entend l'équilibre glycémique d'un individu caractéristique d'une glycémie normale. L'équilibre glycémique à jeun normale chez l'homme est comprise entre environ 0,70 g/L et environ 1,10 g/L (soit entre 70 mg/dL et 110 mg/dL). Une glycémie en dessous de 0,70 g/L caractérise une hypoglycémie alors qu'une glycémie au-dessus de 1,10 g/L mais en dessous de 1,26 g/L caractérise une hyperglycémie modérée pouvant révéler une intolérance au glucose.

Par « **sel d'O-acétylsérine** », on entend un sel d'O-acétylsérine préparé avec des acides ou des bases physiologiquement acceptables. Toutefois, des acides ou des bases utiles, par exemple, pour la purification ou l'isolation de l'OAS peuvent également être utilisés. A titre de sel d'O-acétylsérine, on peut mentionner l'hydrochloride d'O-acétyl-L-sérine.

Par « **dérivé d'O-acétylsérine** », on entend une O-acétylsérine physiologiquement acceptable qui a subi une ou plusieurs modifications, telles que des substitutions ou des migrations intramoléculaires. A titre illustratif de modification, et en particulier de substitution, d'un dérivé d'O-acétylsérine peut être mentionnée la substitution d'au moins l'un des hydrogènes du groupement amine de l'OAS pour former une fonction amide. A titre illustratif, une modification, et en particulier une substitution, additionnelle ou alternative, d'un dérivé d'O-acétylsérine peut être la substitution du groupement méthyle de l'OAS par un groupement alkyl, en particulier par un (C₁-C₆)alkyl, le groupement alkyle étant optionnellement substitué, en particulier avec un halogène, en particulier choisi parmi F and Cl. A titre illustratif, une modification, et en particulier une substitution, additionnelle ou alternative, d'un dérivé d'O-acétylsérine peut être la substitution du proton du groupe carboxyle -COOH de l'OAS pour former une fonction ester. Les dérivés d'OAS sont des OAS qui présentent la même activité, en particulier la même activité pour prévenir et/ou traiter une intolérance au glucose et/ou une maladie associée à une intolérance au glucose chez un individu, que l'OAS. A titre de dérivé d'O-acétylsérine, on peut mentionner la N-acétylsérine.

Tels qu'utilisés ici, les termes « **quantité thérapeutiquement efficace** » et « **quantité prophylactiquement efficace** » se réfèrent à une quantité qui fournit un avantage thérapeutique dans le traitement, la prévention ou la gestion des processus pathologiques considérés. La quantité spécifique qui est thérapeutiquement efficace peut être facilement déterminée par un médecin et peut varier en fonction de facteurs tels que le type et le stade des processus pathologiques considérés, les antécédents médicaux, le sexe, le poids et l'âge du patient, son régime alimentaire, et l'administration d'autres agents thérapeutiques.

Au sens de l'invention, « **significativement** » utilisé dans le contexte d'un changement, signifie que le changement observé est notable ou qu'il a une signification statistique.

Au sens de l'invention les expressions « **substantiellement similaire** », « **substantiellement pas différent** », « **sensiblement similaire** » ou « **sensiblement pas différent** » (ou toute autre variante) utilisées en lien avec une caractéristique de l'invention vise à définir un ensemble de modes de réalisation de l'invention en lien avec cette caractéristique qui sont largement mais pas totalement identiques aux modes de réalisation incluant cette caractéristique.

Par « **test oral de tolérance au glucose** » ou « **test OGTT** », on entend désigner un essai permettant de mesurer la capacité de l'organisme à utiliser le glucose. Un test OGTT est bien connue de l'homme du métier. On peut par exemple utiliser le protocole décrit notamment dans Nagy et al., Study of In Vivo Glucose Metabolism in High-fat Diet-fed Mice Using Oral Glucose Tolérance Test (OGTT) and Insulin Tolérance Test (ITT). J. Vis. Exp. (131), e56672, doi: 10.3791/56672 (2018).

Au sens de l'invention, les termes « **traiter** », « **traitement** », « **thérapie** » ou « **thérapeutique** » se réfèrent à l'administration ou la consommation d'un actif, l'O-acétylsérine ou l'un de ses sels ou de ses dérivés, ou d'une composition comprenant un tel actif à des fins curatives, de soulagement, de réduction, d'atténuation, ou d'amélioration d'une maladie ou d'un désordre pathologique, ou d'un ou plusieurs symptômes associés, ou pour prévenir ou ralentir la progression de ce ou ces symptômes ou de cette maladie, ou pour arrêter le développement de ce ou ces symptômes, ou de cette maladie ou de ce désordre pathologique d'une manière statistiquement significative. Plus particulièrement, « traiter » ou « traitement » incluent toute approche pour obtenir un effet bénéfique ou un résultat désiré à l'égard d'un état d'intolérance au glucose chez un individu. Les résultats cliniques bénéfiques ou souhaités peuvent inclure, mais sans s'y limiter, l'atténuation ou l'amélioration de l'intolérance au glucose, d'une maladie associée à l'intolérance au glucose, telle que le prédiabète ou le diabète de type 2, ou d'un ou de plusieurs symptômes d'une telle maladie ; la diminution ou la réduction de l'étendue de l'intolérance au glucose, d'une maladie associée à l'intolérance au glucose, ou d'un ou de plusieurs symptômes d'une telle maladie ; la stabilisation, c'est-à-dire l'absence d'aggravation, de l'intolérance au glucose, d'une maladie associée à l'intolérance au glucose, ou d'un ou de plusieurs symptômes d'une telle maladie ; la prévention de l'intolérance au glucose, d'une maladie associée à l'intolérance au glucose, ou d'un ou de plusieurs symptômes d'une telle maladie ; la prévention de la propagation d'une maladie associée à l'intolérance au glucose, ou d'un ou de plusieurs symptômes d'une telle maladie ; le ralentissement d'une maladie associée à l'intolérance au glucose, ou d'un ou de plusieurs symptômes d'une telle maladie ou de la progression d'un ou des symptômes d'un telle maladie ; la diminution de la réapparition de l'intolérance au glucose, d'une maladie associée à l'intolérance au glucose, ou d'un ou de plusieurs symptômes d'une telle maladie ; et l'interruption de l'intolérance au glucose, d'une maladie associée à l'intolérance au glucose, ou d'un ou de plusieurs symptômes d'une telle maladie. En d'autres termes, le « traitement » tel qu'utilisé ici comprend toute guérison, amélioration, réduction ou interruption de l'intolérance au glucose, d'une maladie associée à l'intolérance au glucose, ou d'un ou de plusieurs symptômes d'une telle maladie. Une « **réduction** » d'un symptôme ou d'une maladie signifie une diminution de la gravité ou de la fréquence de la maladie ou du symptôme, ou l'élimination de la maladie ou du symptôme. Tel qu'utilisé dans cette description et les revendications, les formes singulières « **un** », « **une** », « **le** » et « **la** » incluent la pluralité, sauf indication contraire et explicite du contexte. L'expression « **véhicule physiologiquement acceptable** » vise à désigner toute substance ou composition compatible avec l'organisme de l'individu auquel un actif de l'invention doit être administré. En particulier, un véhicule physiologiquement acceptable est une substance ou composition dont l'administration à un individu ne s'accompagne pas d'effets délétères significatifs. Il peut s'agir, par exemple, d'un solvant non toxique tel que l'eau ou une solution aqueuse saline. En particulier, un tel véhicule est compatible avec une administration orale ou rectale, et de préférence est adapté à une administration par la voie orale.

La liste des sources, ingrédients et composants indiqués ci-après sont compris comme étant décrits de telle sorte que toutes combinaisons et mélanges de ceux-ci sont également envisagés dans le cadre de la présente invention.

Il est entendu que chaque limitation numérique maximale donnée dans la description comprend chaque limitation numérique inférieure, comme si ces limitations numériques inférieures étaient expressément écrites. Chaque limitation numérique minimale donnée dans cette description comprend toute limitation numérique supérieure, comme si ces limitations numériques supérieures étaient expressément écrites ici. Chaque plage numérique donnée tout au long de la description comprend chaque plage numérique plus étroite incluse dans une telle plage numérique plus large, comme si ces plages numériques plus étroites étaient toutes expressément écrites.

Toutes les listes indiquées dans la description, telles que, par exemple, les listes d'ingrédients, sont destinées et doivent être interprétées comme des groupes Markush. Ainsi, toutes les listes peuvent être lues et interprétées comme des éléments « sélectionnés dans le groupe constitué de » ... liste des éléments ... « et leurs combinaisons et mélanges ».

Il peut être fait référence ci-après à des noms commerciaux de composants comprenant divers ingrédients utilisés dans la présente description. Les inventeurs n'ont pas l'intention d'être limités à des matériaux sous un nom commercial particulier. Des matériaux équivalents (par exemple, ceux obtenus d'une source différente sous un nom ou un numéro de référence différent) à ceux indiqués ici par un nom commercial peuvent être substitués et utilisés dans la description ci-après.

### O-acétylsérine

La présente invention concerne l'O-acétylsérine (OAS), ou l'un de ses sels pour son utilisation dans le traitement et/ou la prévention d'une intolérance au glucose chez un individu. Elle concerne également l'OAS, ou l'un de ses sels pour son utilisation dans le traitement et/ou la prévention d'une ou plusieurs maladies associées à une intolérance au glucose chez un individu.

En particulier, la présente invention concerne l'O-acétylsérine (OAS), ou l'un de ses sels, pour leur utilisation dans le traitement et/ou la prévention d'une intolérance au glucose chez un individu. Elle concerne également l'OAS, ou l'un de ses sels ), pour leur utilisation dans le traitement et/ou la prévention d'une ou plusieurs maladies associées à une intolérance au glucose chez un individu.

L'O-acétylsérine (C₅H₉NO₄) est un métabolite intracellulaire secondaire du métabolisme des acides aminés soufrés de certaines bactéries et plantes de formule suivante :

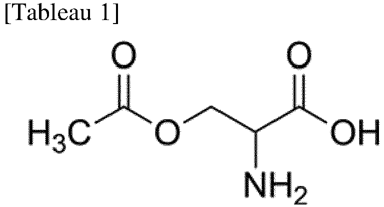

Il s'agit d'un acide α-aminé non protéinogène dérivé de la sérine par acétylation à partir de l'acétyl-CoA sous l'effet de la sérine O-acétyltransférase. C'est un intermédiaire de la biosynthèse de la cystéine chez les bactéries et les plantes, qui la convertissent en cystéine par la cystéine synthase.

Les inventeurs ont identifié l'OAS dans une fraction de surnageant de culture d'une souche de *Streptococcus salivarius* par des analyses en spectrométrie de masse.

Cette espèce commensale est présente en dominance dans la cavité buccale et en sous dominance dans le tractus digestif humain. Les souches de *S. salivarius* sont capables de synthétiser de l'OAS.

L'OAS est aussi présente dans diverses plantes.

Chez l'homme, cette molécule est retrouvée dans la prostate, les urines et le sang et est d'origine bactérienne (bactéries du microbiote intestinal notamment) et alimentaire. L'OAS est référencée HMDB0003011 dans la base des métabolites humains HMDB 4.0 (http://www.hmdb.ca/metabolites/HMDB0003011) et correspond au numéro CAS5147-00-2.

L'O-acétylsérine selon l'invention peut également se trouver sous forme de sel d'OAS. En particulier, l'OAS peut être sous forme d'hydrochloride d'O-acétyl-L-sérine (OAS.HCl). Le sel OAS.HCl (C₅H₉NO₄.HCl) est référencé avec le numéro CAS 66638-22-0.

L'O-acétylsérine peut également se trouver sous forme de dérivé d'OAS ou sous forme d'un sel de dérivé d'OAS.

En particulier, l'OAS peut être sous forme d'une N-acétylsérine (NAS). L'OAS peut également être sous forme d'un sel de NAS.

Une OAS convenant selon l'invention est notamment vendue comme produit chimique sous la référence commerciale A6262_SIGMA^{®} par la société Sigma-Aldrich.

Un dérivé d'OAS, et en particulier la N-acétylsérine, est notamment vendue comme produit chimique sous la référence commerciale A2638^{®} par la société Sigma-Aldrich.

Selon un mode de réalisation particulier, l'O-acétylsérine selon l'invention est un métabolite de synthèse de bactéries, en particulier est un métabolite de synthèse de bactéries du microbiote intestinale, plus particulièrement est un métabolite de synthèse de la souche bactérienne *Streptococcus salivarius.*

Dans certains modes de réalisation, l'OAS ou l'un de ses sels peuvent être inclus dans une composition.

### Composition

La présente invention concerne en outre une composition pour son utilisation dans le traitement et/ou la prévention de l'intolérance au glucose et/ou d'une ou plusieurs maladies associées à l'intolérance au glucose chez un individu comprenant l'O-acétylsérine selon l'invention, ou l'un de ses sels et au moins un véhicule physiologiquement acceptable.

Une composition telle que décrite est administrée à un individu en ayant besoin en une quantité thérapeutiquement efficace. Cette quantité est déterminée en fonction des caractéristiques de chaque individu par des professionnels de la santé.

Dans certains modes de réalisation, la composition est adaptée pour une administration pendant ou en dehors des repas, de préférence en dehors d'un repas. Dans certains modes de réalisation, la composition peut convenir à une administration en au moins une prise journalière, en particulier en une seule prise journalière.

Selon certains modes de réalisation, une composition peut être administrée chaque jour consécutivement sur la période d'administration considérée. Alternativement, une composition peut être administrée sur une période d'un, deux ou plusieurs jours consécutifs suivie d'une période d'interruption d'au moins un, deux ou plusieurs jours consécutifs. Les périodes d'administration et d'interruption peuvent constituer un cycle. L'administration d'une composition peut se faire sur au moins un, voire deux, trois ou plusieurs cycles consécutifs.

Une composition peut être administrée pendant une période d'au moins cinq semaines, ou d'au moins six semaines, ou d'au moins sept semaines ou d'au moins huit semaines, ou d'au moins neuf semaines.

Une composition peut être administrée pendant au moins deux jours consécutifs, en particulier pendant au moins cinq jours consécutifs, sur un intervalle d'une semaine, et sur une période d'au moins une à au moins douze semaines, notamment d'au moins une à au moins dix semaines, notamment d'au moins deux à au moins cinq semaines, et en particulier pendant au moins trois semaines.

Selon certains modes de réalisation, une composition peut être administrée pendant au moins deux jours consécutifs sur un intervalle d'une semaine, en particulier pendant au moins cinq jours consécutifs sur un intervalle d'une semaine.

A titre de véhicule physiologiquement acceptable susceptible d'être mis en oeuvre pour formuler une composition de l'invention, on peut citer, de manière non limitative, l'eau, les tampons salins, notamment un tampon phosphate, le bicarbonate de sodium, les jus, les produits laitiers, notamment le lait ou les yahourts, les compositions alimentaires infantiles, les agents épaississants, tels que le glycérol monostéarate, les agents édulcorants, les agents d'enrobage, tels que l'huile de colza ; l'huile de soja ; l'huile de cacahuète ; la lécithine de soja ou la gélatine de poisson, les agents diluants, tels que le lactose ; le lactose monohydraté ou l'amidon, les liants, tels que la povidone ; l'amidon gélatinisé ; les gommes ; le saccharose ; le polyéthylène glycol (PEG) 4000 ou PEG 6000, les agents dispersants, tels que la cellulose microcrystalline ou l'amidon carboxyméthyl sodique, tel que l'amidon carboxymethyl sodique de type A, les agents lubrifiants, tels que le stéarate de magnésium, les agents fluidifiants, tels que la silice colloïde anhydre, etc.

Un véhicule physiologiquement acceptable peut être n'importe quelle substance utilisée pour la préparation de formes galéniques pharmaceutiques, y compris les matériaux d'enrobage, les matériaux filmogènes, les charges, les agents de désintégration, les matériaux modifiant la libération, les matériaux supports, les diluants, agents liants et autres adjuvants. Les véhicules physiologiquement acceptables habituels comprennent des substances comme le saccharose, le mannitol, le sorbitol, l'amidon et les dérivés d'amidon, le lactose, des agents lubrifiants tels que le stéarate de magnésium, des agents de délitement, et des agents tampons. Également, les véhicules physiologiquement acceptables appropriés comprennent, par exemple, l'eau, les solutions salines, les alcools, les huiles, de préférence les huiles végétales, les polyéthylèneglycols, la gélatine, le lactose, l'amylose, le stéarate de magnésium, les tensioactifs, l'huile de parfum, l'acide gras, les monoglycérides et diglycérides, l'hydroxyméthylcellulose, la polyvinylpyrrolidone et analogues. Les compositions pharmaceutiques peuvent comprendre des agents auxiliaires, comme des lubrifiants, des conservateurs, des stabilisants, des agents mouillants, des émulsifiants, des sels pour influencer la pression osmotique, des tampons, des colorants, des aromatisants et/ou des substances aromatiques.

Les formes liquides sont envisagées pour la présente invention. Celles-ci peuvent comprendre des émulsions, des solutions, des suspensions et des sirops. Les formes solides sont également envisagées, telles que les suppositoires, des comprimés, des pastilles, des pilules, des gélules, des poudres, des formulations effervescentes, des dragées, des granulés, ou des capsules.

Une composition selon l'invention peut comprendre en outre tout additif ou excipient habituellement mis en oeuvre dans le domaine au regard de l'usage considéré pour la composition. Ainsi, une composition de l'invention peut en outre comprendre au moins un choisi parmi un édulcorant, un stabilisant, un antioxydant, un additif, un agent aromatisant et/ou un colorant. La formulation d'une composition de l'invention est effectuée au moyen des procédés usuels mis en oeuvre dans le domaine, notamment pour produire des comprimés dragéifiés, des gélules, des gels, des hydrogels pour libération contrôlée, des émulsions, des mousses, des sirops, des liquides, des comprimés, des capsules, ou des suppositoires.

La présente composition peut être adaptée à une administration par voie orale, sublinguale, nasale ou rectale, en particulier par voie orale.

Dans un mode de réalisation, la composition peut se présenter sous la forme d'un complément alimentaire, d'un complément pour boisson, d'un produit nutritionnel, d'un aliment médical ou d'une composition nutraceutique.

Une composition peut également être sous la forme d'une composition nutritionnelle ou nutraceutique.

En particulier, une composition peut être un complément alimentaire.

Un complément alimentaire peut notamment se présenter sous formes de capsules, gélules, capsules molles, comprimés, comprimés dragéifiés, pilules, pâtes, pastilles, gommes, solutions ou émulsions buvables, sirop ou gel.

Une composition convenant à l'invention peut notamment être formulée sous forme d'un complément alimentaire choisi parmi des produits laitiers, des boissons laitières, des yaourts, des jus de fruits ou de légumes ou leurs concentrés, des poudres, des boissons à base de soja ou de céréales, des céréales de petit déjeuner telles que flocons de muesli, des poudres de jus de fruits et de légumes, des barres de céréales et/ou de chocolat, des confiseries, des pâtes à tartiner, des farines de lait, des smoothies, des glaces, des produits à base de fruits reconstitués, des barres alimentaires, des sauces, des compléments sportifs, y compris les compléments sportifs laitiers et non laitiers, un dessert, un aliment congelé, une soupe, une suspension liquide, un comprimé, une gomme ou un bonbon.

Avantageusement, une composition selon la présente invention, prévue pour une administration par la voie orale, peut être pourvue d'un enrobage résistant au suc gastrique, afin d'assurer que l'O-acétylsérine de l'invention comprise dans ladite composition puisse traverser l'estomac sans être endommagé. La libération de l'OAS selon l'invention peut ainsi se produire pour la première fois dans le tractus intestinal supérieur.

Selon un mode de réalisation particulier, une composition pour voie orale convenant à l'invention peut être choisie parmi une poudre, des granulés, un produit alimentaire, une boisson, un produit pharmaceutique, un nutraceutique, un additif alimentaire, un complément alimentaire, une capsule, et une gélule.

Dans un autre mode de réalisation de l'invention, une composition peut être administrée par voie rectale. En particulier, une composition pour voie rectale peut être préparée sous la forme d'un suppositoire, un lavement ou une mousse.

Dans un mode de réalisation, une composition peut comprendre en outre au moins un actif additionnel choisi parmi : des métabolites, des antioxydants, des huiles de poisson, DHA, EPA, des vitamines, des minéraux, des phytonutriments, une protéine, un lipide, des probiotiques et des combinaisons de ceux-ci.

A titre d'actifs probiotiques susceptibles d'être mise en oeuvre de manière additionnelle on peut citer les souches de bactéries probiotiques issues de *Streptococcus, Lactobacillus, Lactococcus, Bifidobacterium, Veillonella, Desemzia, Coprococcus, Collinsella, Citrobacter, Turicibacter, Sutterella, Subdoligranulum, Sporobacter, Sporacetigenium, Ruminococcus, Roseburia, Proteus, Propionobacterium, Leuconostoc, Weissella, Pediococcus, Prevotella, Parabacteroides, Papillibacter, Oscillospira, Melissococcus, Dorea, Dialister, Clostridium, Cedecea, Catenibacterium, Butyrivibrio, Buttiauxella, Bulleidia, Bilophila, Bacteroides, Anaerovorax, Anaerostopes, Anaerofilum, Enterobacteriaceae, Fermicutes, Atopobium, Alistipes, Acinetobacter, Slackie, Shigella, Shewanella, Serratia, Mahella, Lachnospira, Klebsiella, Idiomarina, Fusobacterium, Faecalibacterium, Eubacterium, Enterococcus, Enterobacter,* ou *Eggerthella.*

A titre de phytonutriments convenant à l'invention, on peut mentionner les caroténoïdes, les polyphénols ou le resvératrol. A titre d'antioxydants convenant à l'invention, on peut mentionner la vitamine C, le glutathion, la caféine, ou le tocophérol. A titre de vitamines convenant à l'invention, on peut citer les vitamines A, B, C, ou D. A titre de minéraux, on peut citer le fer, le magnésium, le calcium, le zinc, le cuivre, ou le sodium.

En particulier, la composition peut comprendre au moins un actif additionnel permettant de prévenir et/ou traiter une intolérance au glucose. Ledit au moins un actif additionnel permettant de prévenir et/ou traiter une intolérance au glucose est différent de l'OAS, ou l'un de ses sels.

Selon un mode de réalisation, une composition selon l'invention comprend l'O-acétylsérine, ou l'un de ses sels, selon l'invention, au moins un véhicule physiologiquement acceptable, et au moins un actif additionnel permettant de prévenir et/ou traiter une intolérance au glucose.

En particulier, l'actif additionnel peut être toute molécule antidiabétique et en particulier la metformine. Une intolérance au glucose peut être traitée avec un inhibiteur d'alpha-glucosidase (Acarbose, Voglibose), ou un inhibiteur de la lipase pancréatique (Orlistat), ou un glinide (Nateglinide) ou un incrétino-mimétique (Liraglutide).

La metformine (C₄H₁₁N₅) est un antidiabétique oral de la famille des biguanides normoglycémiants utilisé dans le traitement du diabète de type 2 et conseillée pour des personnes prédiabétiques les plus à risque de développer un diabète de type 2.

Selon un aspect, l'administration dans une même composition de l'OAS, ou l'un de ses sels, et de la metformine permet de réduire la quantité de metformine dans la composition, par rapport à une composition comprenant uniquement de la metformine, tout en ayant une action équivalente sur la prévention et/ou le traitement d'une intolérance au glucose et/ou d'une maladie associée à une intolérance au glucose.

Selon un mode de réalisation particulier, la composition n'est pas une composition nutritionnelle comprenant un mélange d'oligosaccharides consistant en un oligosaccharide N-acétylé, un galacto-oligosaccharide et un oligosaccharide sialylé.

Selon un autre mode de réalisation, une composition de l'invention n'est pas destinée à être utilisée pour réduire et/ou éviter une accumulation excessive de masse adipeuse chez un nourrisson ou un jeune enfant, et/ou pour la prévention d'un trouble de santé à un âge ultérieur lié à une accumulation excessive de masse adipeuse chez un nourrisson ou un jeune enfant, comme l'obésité à un âge ultérieur, et les comorbidités associées.

### Intolérance au glucose et maladies associées

Comme indiqué précédemment, l'OAS selon l'invention, ou l'un de ses sels ou une composition selon l'invention, sont mis en oeuvre dans une méthode thérapeutique pour prévenir et/ou traiter une intolérance au glucose et/ou une ou plusieurs maladies associées à une intolérance au glucose chez un individu.

Un individu ou patient considéré selon l'invention peut être un mammifère. Un mammifère visé par l'invention peut être, par exemple, choisi parmi des animaux domestiques (tels que les bovins, les ovins, les chats, les chiens, et les chevaux, notamment les chats et les chiens, les primates, tels que les primates humains et non-humains, les lapins, et les rongeurs, tels que les souris et les rats. Selon un mode de réalisation, un individu ou patient visé par l'invention peut être un être humain.

L'OAS ou l'un de ses sels ou une composition selon l'invention, peuvent convenir en particulier pour une utilisation dans un traitement thérapeutique pour prévenir et/ou traiter un prédiabète, et/ou une maladie ou un désordre associé au prédiabète, ou un symptôme du prédiabète.

Une intolérance au glucose ou un prédiabète peuvent être asymptomatique. Cependant, ils peuvent être associés à une obésité, une dyslipidémie avec un taux élevé de triglycérides et/ou un faible taux de cholestérol HDL, et de l'hypertension. L'intolérance au glucose et le prédiabète sont associés à un risque augmenté de maladies cardiovasculaires. Le prédiabète et l'intolérance au glucose sont considérés comme une phase précoce du diabète de type 2. Une intolérance au glucose ou un prédiabète peut être diagnostiqué par différentes méthodes connues par l'homme du métier. Notamment, une intolérance au glucose chez l'homme est diagnostiquée sur la base de deux critères :
- la glycémie à jeun, et
- la glycémie deux heures après une ingestion orale de glucose.

Ainsi, une intolérance au glucose peut être diagnostiquée si :(i) à jeun, la glycémie est : comprise entre 6.1 et 6.9 mmol/L (110-125 mg/dL) et si (ii) deux heures après une ingestion de glucose oral de 75 g, la glycémie est comprise entre 7,8 mmol/L à 11,0 mmol/L (140 mg/dL à 199 mg/dL).

La mesure de la glycémie deux heures après une ingestion de glucose est réalisée par un test oral de tolérance au glucose ou test OGTT. Un tel test est décrit dans Hjellestad et al., HbA1c versus oral glucose tolérance test as a method to diagnose diabètes mellitus in vascular surgery patients, Cardiovascular Diabetology, 12:79 (2013).

L'intolérance au glucose ou le prédiabète peuvent être généralement suivie d'un diabète de type 2. Le diabète de type 2 (ou DT2) est une maladie associée à une intolérance au glucose ou au prédiabète.

Selon un mode de réalisation, l'OAS ou un de ses sels ou de ses dérivés ou une composition selon l'invention peuvent convenir en particulier pour une utilisation dans un traitement thérapeutique pour prévenir et/ou traiter un diabète de type 2.

Un DT2 peut être diagnostiqué par différentes méthodes connues par l'homme du métier. Notamment, un DT2 peut être diagnostiqué si un ou plusieurs critères sont satisfaits parmi :
- la glycémie à jeun,
- la glycémie deux heures après une gestion de glucose oral, ou
- la glycémie post-prandiale ou aléatoire
- l'hémoglobine glyquée A1C (HbA1C).

Ainsi, un DT2 est diagnostiqué si , la glycémie est : (i) à jeun, supérieure ou égale à un 7,0 mmol/L (126 mg/dL), ou si (ii) deux heures après une ingestion de glucose oral de 75 g, supérieure ou égale à 11,1 mmol/L (200 mg/dL), ou si (iii) la glycémie plasmatique aléatoire est supérieure à 11,1 mmol/L (200 mg/dL) ou si (iv) le taux d'hémoglobine glyquée A1c (HbA1c) est supérieure ou égale à 48 mmol/mol (équivalent à 6,5 %).

Selon un mode de réalisation, la présente invention concerne également une méthode de traitement thérapeutique pour prévenir et/ou traiter une intolérance au glucose et/ou une maladie associée à une intolérance au glucose chez un individu en ayant besoin comprenant au moins une étape d'administration audit individu d'OAS ou l'un de ses sels ou d'administration audit individu d'une composition selon l'invention.

Selon un mode de réalisation, la présente invention concerne également une méthode de traitement thérapeutique pour prévenir et/ou traiter un prédiabète et/ou une maladie associée au prédiabète chez un individu en ayant besoin comprenant au moins une étape d'administration audit individu d'OAS ou l'un de ses sels, ou d'administration audit individu d'une composition selon l'invention.

Selon un mode de réalisation, la présente invention concerne également une méthode de traitement thérapeutique pour prévenir et/ou traiter un diabète de type 2 chez un individu en ayant besoin comprenant au moins une étape d'administration audit individu d'OAS ou l'un de ses sels ou d'administration audit individu d'une composition selon l'invention.

Une méthode selon l'invention peut comprendre, préalablement à l'étape d'administration d'un actif de l'invention, une étape de diagnostic de l'intolérance au glucose, du prédiabète ou du diabète de type 2. Un tel diagnostic peut être réalisé par toute méthode diagnostic connue de l'homme de l'art pour cet effet, et notamment par une des méthodes décrites ci-dessus.

Une méthode selon l'invention peut comprendre en outre une étape d'observation de la réduction, suppression, ou amélioration d'un symptôme ou d'un marqueur glycémique de l'intolérance au glucose, du prédiabète ou d'un diabète de type 2.

Selon un mode de réalisation particulier, un individu ou un patient considéré pour l'invention peut être en surpoids ou obèse.

Une utilisation ou une méthode selon l'invention peut comprendre l'administration à un individu ou patient en ayant besoin d'OAS ou l'un de ses sels ou d'une composition selon l'invention par toute voie d'administration susceptible de convenir. On peut par exemple citer une administration par voie orale, sublinguale, nasale, ou rectale. De préférence, une administration d'OAS ou l'un de ses sels, ou d'une composition selon l'invention peut se faire par voie orale.

Selon un mode de réalisation, l'OAS ou l'un de ses sels ou la composition selon l'invention peut être administrée en au moins une prise journalière, en particulier deux ou trois prises journalières. Plus particulièrement, en au moins une prise journalière.

La prise d'OAS ou l'un de ses sels ou de la composition selon l'invention peut se faire au moment d'un repas, ou dehors des repas. Sur une période de 24 h, la prise d'OAS ou l'un de ses sels ou d'une composition selon l'invention peut se faire à tout moment. En particulier, la prise d'OAS ou l'un de ses sels ou d'une composition selon l'invention peut se faire le matin, et en particulier au moment du repas du matin.

En particulier, l'administration d'OAS ou l'un de ses sels ou d'une composition selon l'invention peut se faire en dehors des repas, notamment en une prise journalière.

La période de temps pendant laquelle l'OAS ou l'un de ses sels ou d'une composition selon l'invention est administrée dépend de plusieurs facteurs, tels que l'âge, le poids, le sexe du patient, la présence d'autres désordres pathologiques, le régime alimentaire, et est adaptée par l'homme de l'art selon la pratique usuelle dans le domaine.

Une période de temps convenant pour une administration d'OAS ou l'un de ses sels ou d'une composition selon l'invention peut être d'au moins cinq semaines, ou d'au moins six semaines, ou d'au moins sept semaines ou d'au moins huit semaines, ou d'au moins neuf semaines.

Durant une période d'administration, l'OAS ou l'un de ses sels ou la composition selon l'invention peut être administrée chaque jour consécutivement, ou l'administration peut être réalisée par période d'un ou plusieurs jours, suivie d'une période d'interruption d'un ou plusieurs jours, le cas échéant suivie d'un ou plusieurs cycles d'administration et d'interruption.

Selon certains modes de réalisation, l'OAS ou l'un de ses sels ou la composition selon l'invention peuvent être administrées sur une période d'un, deux ou plusieurs jours consécutifs suivie d'une période d'interruption d'au moins un, deux ou plusieurs jours consécutifs. Les périodes d'administration et d'interruption peuvent constituer un cycle. L'administration d'OAS ou l'un de ses sels ou d'une composition selon l'invention peut se faire sur au moins un, voire deux, trois ou plusieurs cycles consécutifs.

Ainsi, l'OAS ou l'un de ses sels ou la composition selon l'invention peuvent être administrés pendant au moins deux jours consécutifs sur un intervalle d'une semaine. Selon un mode de réalisation, l'administration peut être réalisée pendant au moins cinq jours consécutifs sur un intervalle d'une semaine. L'administration peut être poursuivie sur deux, trois ou plusieurs semaines comme indiqué ci-dessus.

Selon un mode de réalisation, l'OAS ou l'un de ses sels ou la composition selon l'invention peuvent être administrés en une quantité ou dose thérapeutique efficace. En particulier, l'OAS ou l'un de ses sels ou la composition selon l'invention peuvent être administrés à une dose journalière équivalente à une dose variant d'au moins environ 70 mg/kg/j à environ au moins 2000 mg/kg/j. En particulier, une dose convenant à l'invention peut être d'au moins environ 100 mg/kg/j à au moins environ 1000 mg/kg/j, et en particulier d'au moins environ 200 mg/kg/j à au moins environ 500 mg/kg/j.

Selon un autre aspect, l'invention concernent également l'utilisation de l'OAS ou l'un de ses sels ou la composition selon l'invention pour la fabrication d'un médicament. En particulier, elle concerne l'utilisation de l'OAS ou l'un de ses sels ou la composition selon l'invention pour la fabrication d'un médicament permettant de prévenir et/ou traiter une intolérance au glucose et/ou une ou plusieurs maladies associées à une intolérance au glucose.

Il est entendu que l'invention englobe toutes les variantes, combinaisons et permutations dans lesquelles une ou plusieurs limitations, éléments, clauses, termes descriptifs, etc., d'une ou plusieurs des revendications énumérées ci-après peuvent être introduites dans une autre revendication dépendante de la même revendication de base (ou, selon le cas, toute autre revendication), sauf indication contraire ou à moins qu'il ne soit évident pour l'homme du métier qu'une contradiction ou une incohérence se produirait. Lorsque des éléments sont présentés sous forme de listes (par exemple, dans le groupe Markush ou un format similaire), il faut comprendre que chaque sous-groupe d'éléments est également divulgué, et tout élément peut être supprimé du groupe. Il doit être entendu qu'en général, lorsque l'invention ou des aspects de l'invention est/sont désignés comme comprenant des éléments, des caractéristiques, etc., particuliers, certains modes de réalisation de l'invention ou de certains aspects de l'invention consistent, ou consistent essentiellement en de tels éléments, caractéristiques, etc. Pour des raisons de simplicité, ces modes de réalisation n'ont pas toujours été spécifiquement énoncés en autant de mots. Il doit également être entendu que tout mode de réalisation ou aspect de l'invention peut être explicitement exclu des revendications, indépendamment du fait que l'exclusion spécifique soit mentionnée dans la spécification. Les publications et autres documents cités pour décrire l'arrière-plan de l'invention et pour fournir des détails supplémentaires concernant sa mise en oeuvre sont incorporés ici par référence.

L'invention est décrite ci-dessous de façon plus détaillée au moyen des exemples suivants qui sont présentés à titre d'illustration uniquement, et ne sont pas limitatifs de l'invention.

L'invention est décrite ci-dessous de façon plus détaillée au moyen des exemples suivants qui sont présentés à titre d'illustration uniquement.

### Exemples

### Exemple 1 : action préventive d'un traitement à base d'un actif selon l'invention sur des individus sains

Des souris mâles C57BL/6JRj âgées de 7 semaines de chez Janvier Labs ont été nourries avec un régime alimentaire classique utilisé en animalerie.

Un premier groupe de 10 souris, n'ayant pas reçu de traitement à l'OAS, a servi de groupe contrôle.

Un second groupe de 10 souris a été traité avec une dose journalière d'OAS de 1000 mg/kg, pendant 5 jours. L'OAS qui a été utilisé est le produit commercialisé sous le nom A6262-SIGMA par la société Sigma-Aldrich (CAS N°66638-22-0). Ce groupe est le groupe traitement OAS.

Un test de tolérance oral au glucose (OGTT) a été fait sur chacun des deux groupes de souris. Ce test consiste à suivre les concentrations plasmatiques en glucose pendant 120 min, après que les souris aient reçu une charge orale de glucose. Ainsi, à T0 (Glycémie à jeun), du glucose (2.5g/kg) est donné par voie orale à l'animal. Une goutte de sang est prélevée au niveau de la queue toutes les 15 à 30 minutes pendant 90 minutes afin de mesurer la glycémie avec un glucomètre à bandelettes (Accu-Check Perfoma, disponible en pharmacie).

L'OGTT a été réalisé au 5ème jour de traitement 2h après que le groupe traitement OAS ait reçu une dose de 500 mg/kg. Au moment du test OGTT, les souris du groupe traitement reçoivent également 500 mg/kg d'OAS en même temps que la charge orale de glucose.

Le groupe contrôle n'a reçu que la charge orale de glucose.

La glycémie à jeun des animaux tend à être plus faible dans le groupe traitement OAS et la glycémie à 15min, 30min et 60min après la charge orale de glucose est significativement plus faible dans le groupe traitement OAS comparé au groupe contrôle.

Les résultats de ce test montrent qu'un traitement avec l'OAS de 5 jours diminue la glycémie à jeun du groupe traité et diminue significativement l'hyperglycémie provoquée par une charge orale en glucose (Figure 4).

Ce traitement diminue significativement l'hyperglycémie avec une aire sous la courbe plus faible que celle du groupe contrôle (Figure 4), démontrant l'effet de l'O-acétylsérine sur la régulation de l'hyperglycémie postprandiale et ainsi l'action préventive d'un actif selon l'invention dans l'établissement d'une résistance à l'insuline et/ou à d'un prédiabète.

### Exemple 2 : action préventive d'une dose unique d'un actif selon l'invention sur des individus sains sur la glycémie postprandiale

Des souris mâles C57BL/6JRj âgées de 7 semaines de chez Janvier Labs ont été nourries avec un régime alimentaire classique utilisé en animalerie.

Un premier groupe de 5 souris, n'ayant pas reçu de traitement à l'OAS, a servi de groupe contrôle.

Un second groupe de 5 souris a été traité avec une dose unique d'OAS de 1000 mg/kg. L'OAS qui a été utilisé est le produit commercialisé sous le nom A6262_SIGMA par la société Sigma-Aldrich (CAS N°66638-22-0). Ce groupe est le groupe traitement OAS.

Un test de tolérance oral au glucose (OGTT) a été fait sur chacun des deux groupes de souris. Ce test consiste à suivre les concentrations plasmatiques en glucose pendant 120 min, après que les souris aient reçu une charge orale de glucose. Ainsi, à T0 (Glycémie à jeun), du glucose (2.5g/kg) est donné par voie orale à l'animal. Une goutte de sang est prélevée au niveau de la queue toutes les 30 minutes pendant 120 minutes afin de mesurer la glycémie avec un glucomètre à bandelettes (Accu-Check Perfoma, disponible en pharmacie).

Lors du test OGTT le groupe traitement OAS a reçu une dose de 1000 mg/kg d'OAS en même temps que la charge orale de glucose. Le groupe contrôle n'a reçu que la charge orale de glucose. La glycémie à 15min après la charge orale de glucose est significativement plus faible dans le groupe traitement OAS comparé au groupe contrôle.

Les résultats de ce test montrent qu'après 15 minutes, une seule dose d'OAS diminue significativement l'hyperglycémie provoquée par une charge orale en glucose (Figure 5). Ce traitement diminue significativement l'hyperglycémie avec une aire sous la courbe plus faible que celle du groupe contrôle (Figure 5). Une dose unique d'O-acétylsérine est capable de diminuer l'hyperglycémie postprandiale. Cela démontre ainsi l'action préventive d'une dose unique d'un actif selon l'invention dans l'établissement d'une résistance à l'insuline et/ou à d'un prédiabète.

### Exemple 3 : action de l'actif selon l'invention sur des individus HFD

L'action d'un actif selon l'invention a également été testé chez des individus sous régime obésogène.

Des souris mâles C57BL/6JRj âgées de 7 semaines de chez Janvier Labs ont été nourries avec un régime alimentaire riche en graisses (High Fat Diet - HFD, 45% lipides et 20% saccharose) pendant 12 semaines. Ce régime est comparable à un régime alimentaire obésogène favorisant l'intolérance au glucose.

Un premier groupe de 10 souris ayant reçu cette alimentation a été traité avec une dose journalière d'OAS de 200 mg/kg/j, à une fréquence de 5 jours par semaine pendant les 12 semaines de régime. L'OAS qui a été utilisé est le produit commercialisé sous le nom A6262-SIGMA par la société Sigma-Aldrich (CAS N° 66638-22-0). Ce groupe est le groupe HFD+OAS.

Le second groupe de 9 souris ayant reçu un régime alimentaire HFD n'a pas reçu de traitement OAS. C'est le groupe HFD.

Un dernier groupe de 9 souris, n'ayant pas reçu de régime alimentaire particulier ni de traitement à l'OAS, a servi de groupe contrôle.

Chez les souris sous régime HFD, une prise de poids significativement plus élevée (*p<0.05) et une augmentation significative de l'adiposité au niveau de l'épididyme des souris (****p<0.0001) ont été observées après 12 semaines de régime, par rapport aux souris n'ayant pas été nourries avec un régime HFD (groupe contrôle). Ces caractéristiques sont le signe d'une installation de l'obésité chez les souris (Figure 1).

Un test de tolérance oral au glucose (OGTT) a été fait sur chacun des trois groupes de souris. Ce test consiste à suivre les concentrations plasmatiques en glucose pendant 120 min, après que les souris aient reçu une charge orale de glucose. Ainsi, à T₀ (Glycémie à jeun), du glucose (2g/kg) est donné par voie orale à l'animal. Une goutte de sang est prélevée au niveau de la queue toutes les 30 minutes pendant 120 minutes afin de mesurer la glycémie avec un glucomètre à bandelettes (Accu-Check Perfoma, disponible en pharmacie).

La tolérance au glucose est le reflet de l'organisme à réguler le retour à une glycémie normale après une charge en glucose.

L'OGTT a été fait à 5 semaines et à 9 semaines après le début du régime alimentaire obésogène. Les résultats de ce test montrent l'installation d'une intolérance au glucose dès 5 semaines chez les groupes de souris ayant un régime alimentaire HFD. Le traitement avec l'OAS n'a pas d'effet visible dans le groupe HFD + OAS après 5 semaines (Figure 2). Cependant, après 9 semaines, ce traitement diminue significativement l'intolérance au glucose avec une aire sous la courbe similaire à celle du groupe contrôle (Figure 3).

En conclusion, un traitement avec l'O-acétylsérine permet de réguler *in vivo* le métabolisme du glucose avec notamment une amélioration de l'intolérance au glucose associée à un régime obésogène.

## Revendications

1. O-acétylsérine, ou l'un de ses sels pour son utilisation dans le traitement et/ou la prévention de l'intolérance au glucose et/ou d'une ou plusieurs maladies associées à une intolérance au glucose chez un individu.

2. O-acétylsérine pour son utilisation selon la revendication 1, dans laquelle la maladie associée à une intolérance au glucose est le diabète de type 2.

3. O-acétylsérine pour son utilisation selon la revendication 1 ou 2, dans laquelle l'individu est en surpoids ou obèse.

4. O-acétylsérine pour son utilisation selon l'une quelconque des revendications précédentes, dans laquelle l'O-acétylsérine est un métabolite de synthèse de bactéries, en particulier est un métabolite de synthèse de bactéries du microbiote intestinale.

5. O-acétylsérine pour son utilisation selon la revendication 4, dans laquelle l'O-acétylsérine est un métabolite de synthèse de la souche bactérienne *Streptococcus salivarius.*

6. Composition pour son utilisation dans le traitement et/ou la prévention de l'intolérance au glucose et/ou d'une ou plusieurs maladies associées à l'intolérance au glucose chez un individu comprenant une O-acétylsérine, ou l'un de ses sels, et un véhicule physiologiquement acceptable.

7. Composition pour son utilisation selon la revendication 6, dans laquelle la maladie associée à une intolérance au glucose est le diabète de type 2.

8. Composition pour son utilisation selon la revendication 6 ou 7, dans laquelle l'individu est obèse.

9. Composition pour son utilisation selon l'une quelconque des revendications 6 à 8, dans laquelle l'O-acétylsérine est un métabolite de synthèse de bactéries, en particulier est un métabolite de synthèse de bactéries du microbiote intestinale.

10. Composition pour son utilisation selon la revendication 9, dans laquelle l'O-acétylsérine est un métabolite de synthèse de la souche bactérienne *Streptococcus salivarius.*

11. Composition pour son utilisation selon l'une quelconque des revendications 6 à 10, dans laquelle la composition est une composition pour voie orale, en particulier sous la forme d'une poudre, de granulés, d'un produit alimentaire, d'une boisson, d'un produit pharmaceutique, d'un nutraceutique, d'un additif alimentaire, d'un complément alimentaire, d'une capsule ou d'une gélule.

12. Composition pour son utilisation selon l'une quelconque des revendications 6 à 11, dans laquelle la composition comprend en outre un actif additionnel, en particulier choisi parmi des métabolites, des antioxydants, des huiles de poisson, DHA, EPA, des vitamines, des minéraux, des phytonutriments, une protéine, un lipide, des probiotiques, des actifs additionnels permettant de prévenir et/ou traiter une intolérance au glucose, et des combinaisons de ceux-ci.

## Patentansprüche

1. 0-Acetylserin oder ein Salz davon zur Verwendung bei der Behandlung und/oder Prävention von Glucoseintoleranz und/oder einer oder mehreren mit Glucoseintoleranz assoziierten Erkrankungen bei einem Individuum.

2. 0-Acetylserin zur Verwendung nach Anspruch 1, wobei es sich bei der mit Glucoseintoleranz assoziierten Erkrankung um Typ-2-Diabetes handelt.

3. 0-Acetylserin zur Verwendung nach Anspruch 1 oder 2, wobei das Individuum übergewichtig oder fettleibig ist.

4. 0-Acetylserin zur Verwendung nach einem der vorhergehenden Ansprüche, wobei es sich bei dem 0-Acetylserin um einen synthetischen Metaboliten von Bakterien, insbesondere einen synthetischen Metaboliten von Bakterien der Darmmikrobiota, handelt.

5. 0-Acetylserin zur Verwendung nach Anspruch 4, wobei es sich bei dem 0-Acetylserin um einen synthetischen Metaboliten des Bakterienstamms *Streptococcus salivarius* handelt.

6. Zusammensetzung zur Verwendung bei der Behandlung und/oder Prävention von Glucoseintoleranz und/oder einer oder mehreren mit Glucoseintoleranz assoziierten Erkrankungen bei einem Individuum, umfassend ein 0-Acetylserin oder ein Salz davon und ein physiologisch unbedenkliches Vehikel.

7. Zusammensetzung zur Verwendung nach Anspruch 6, wobei es sich bei der mit Glucoseintoleranz assoziierten Erkrankung um Typ-2-Diabetes handelt.

8. Zusammensetzung zur Verwendung nach Anspruch 6 oder 7, wobei das Individuum fettleibig ist.

9. Zusammensetzung zur Verwendung nach einem der Ansprüche 6 bis 8, wobei es sich bei dem 0-Acetylserin um einen synthetischen Metaboliten von Bakterien, insbesondere einen synthetischen Metaboliten von Bakterien der Darmmikrobiota, handelt.

10. Zusammensetzung zur Verwendung nach Anspruch 9, wobei es sich bei dem 0-Acetylserin um einen synthetischen Metaboliten des Bakterienstamms *Streptococcus salivarius* handelt.

11. Zusammensetzung zur Verwendung nach einem der Ansprüche 6 bis 10, wobei es sich bei der Zusammensetzung um eine Zusammensetzung zur oralen Verabreichung handelt, insbesondere in Form eines Pulvers, eines Granulats, eines Nahrungsmittelprodukts, eines Getränks, eines pharmazeutischen Produkts, eines Nutrazeutikums, eines Nahrungsmittelzusatzes, eines Nahrungsergänzungsstoffs, einer Kapsel oder einer Gelkapsel.

12. Zusammensetzung zur Verwendung nach einem der Ansprüche 6 bis 11, wobei die Zusammensetzung außerdem einen zusätzlichen Wirkstoff umfasst, der insbesondere aus Metaboliten, Antioxidantien, Fischölen, DHA, EPA, Vitaminen, Mineralien, Phytonährstoffen, einem Protein, einem Lipid, Probiotika, zusätzlichen Mitteln zur Prävention und/oder Behandlung von Glucoseintoleranz und Kombinationen davon ausgewählt ist.

## Claims

1. O-Acetylserine, or one of its salts, for use thereof in the treatment and/or the prevention of glucose intolerance and/or one or more diseases associated with glucose intolerance in an individual.

2. O-Acetylserine for use thereof according to Claim 1, wherein the disease associated with glucose intolerance is type 2 diabetes.

3. O-Acetylserine for use thereof according to Claim 1 or 2, wherein the individual is overweight or obese.

4. O-Acetylserine for use thereof according to any one of the preceding claims, wherein the 0-acetylserine is a synthetic metabolite of bacteria, and in particular is a synthetic metabolite of bacteria of the intestinal microbiota.

5. O-Acetylserine for use thereof according to Claim 4, wherein the 0-acetylserine is a synthetic metabolite of the *Streptococcus salivarius* bacterial strain.

6. Composition for use thereof in the treatment and/or the prevention of glucose intolerance and/or one or more diseases associated with glucose intolerance in an individual, comprising an 0-acetylserine, or one of its salts, and a physiologically acceptable carrier.

7. Composition for use thereof according to Claim 6, wherein the disease associated with glucose intolerance is type 2 diabetes.

8. Composition for use thereof according to Claim 6 or 7, wherein the individual is obese.

9. Composition for use thereof according to any one of Claims 6 to 8, wherein the 0-acetylserine is a synthetic metabolite of bacteria, and in particular is a synthetic metabolite of bacteria of the intestinal microbiota.

10. Composition for use thereof according to Claim 9, wherein the 0-acetylserine is a synthetic metabolite of the *Streptococcus salivarius* bacterial strain.

11. Composition for use thereof according to any one of Claims 6 to 10, wherein the composition is an oral composition, in particular in the form of a powder, granules, a food product, a beverage, a pharmaceutical product, a nutraceutical, a food additive, a food supplement, a capsule or a gel capsule.

12. Composition for use thereof according to any one of Claims 6 to 11, wherein the composition additionally comprises an additional active ingredient, in particular chosen from metabolites, antioxidants, fish oils, DHA, EPA, vitamins, minerals, phytonutrients, a protein, a lipid, probiotics, additional active ingredients for preventing and/or treating glucose intolerance, and combinations of these.
